# NEUE EUROPÄISCHE PATENTSCHRIFT

(11) **EP 0 414 928 B2**
(45) Veröffentlichungstag und Bekanntmachung des Hinweises auf die Entscheidung über den Einspruch: **29.01.2003**
(45) Hinweis auf die Patenterteilung: 02.11.1994
(21) Anmeldenummer: 89115849.5
(22) Anmeldetag: 28.08.1989
(51) Int. Cl.: A61N 1/36, A61N 1/365

(54) **Medizinisches Gerät zur Stimulation eines physiologischen Vorganges eines Lebewesens mit sich selbsttätig an die körperliche Aktivität des Lebewesens anpassender Stimulationsintensiät**
Medical apparatus for stimulating a physiological process in a living being, whereby the stimulation intensity is automatically adapted to body activity
Appareil médical de stimulation d'un processus physiologique dans un corps vivant où l'intensité de stimulation est automatiquement adaptée à l'activité corporelle

(43) Veröffentlichungstag der Anmeldung: 06.03.1991
(73) Patentinhaber: St. Jude Medical AB, 175 84 Järfälla (SE)
(72) Erfinder: Högnelid, Kurt, Dipl.-Ing., S-172 35 Sundbyberg (SE); Engström, Jan, Dipl.-Ing., S-163 55 Spanga (SE)
(74) Vertreter: Harrison, Michael Charles

(56) Entgegenhaltungen:
- EP-A- 0 080 348
- EP-A- 0 249 821
- WO-A-87/01947
- DE-A- 3 709 022

## Beschreibung

Die Erfindung betrifft ein in den Körper eines Lebewesens implantierbares medizinisches Gerät mit Mitteln zum Stimulieren eines physiologischen Vorganges des Lebewesens mit einstellbarer Stimulationsintensität und mit selbsttätigen Stellmitteln zur Anpassung der Stimulationsintensität an die körperliche Aktivität des Lebewesens, die die Stimulationsintensität anhand eines der körperlichen Aktivität des Lebewesens entsprechenden Signals einer Sensoreinrichtung einstellen, wobei die Empfindlichkeit, mit der die Einstellung der Stimulationsintensität anhand des Signals der Sensoreinrichtung erfolgt. verringerbar ist. Der Begriff Stimulationsintensität soll hier umfassend verstanden werden. d.h.. dass die Dauer. die Häufigkeit, die Folgefrequenz. die Amplitude usw., mit der die Mittel zum Stimulieren tätig werden. einzeln oder in Kombination als Mass für die Stimulationsintensität verstanden werden sollen. Der Begriff Empfindlichkeit soll so verstanden werden, dass er angibt. welchen Wert der der körperlichen Aktivität entsprechende Signalparameter des Signals der Sensoreinrichtung, z.B. die Signalamplitude, aufweisen muss. um zur Einstellung einer bestimmten Stimulationsintensität zu führen. Eine Verringerung der Empfindlichkeit bedeutet dann, dass ein höherer Wert des jeweiligen Signalparameter als zuvor vorliegen muss. um zur Einstellung der gleichen Stimulationsintensität zu führen.

Mittels derartiger Geräte soll erreicht werden, dass ein Lebewesen, das auf ein Gerät zur Stimulation eines physiologischen Vorganges angewiesen ist. ein möglichst normales Leben führen kann. Der jeweilige physiologische Vorgang soll also in Abhängigkeit von der körperlichen Aktivität des Lebewesens mit einer Stimulationsintensität stimuliert werden, die derjenigen entspricht, die bei einem gesunden Lebewesen bei gleicher körperlicher Aktivität vorliegen würde. Dies soll sowohl für Zustände hoher körperlicher Aktivität als auch für Ruhezustände gewährleistet sein.

WO-A-87 01947 und DE-A-37 09 022 beschreiben frequenzvariable Herzschrittmacher. die über Sensorsignale gesteuert werden. Gemäß dem Sensorsignal wird die Schnttmacherrate vorgegeben. Bei Scheinbelastungen erhöhen diese Schrittmacher die Rate und starten gleichzeitig einen Timer. Wird eine gewisse Zeitdauer überschritten, so wird in WO-A-8701947 die Rate automatisch abgesenkt. während in DE-A-37 09 022 eine weiterer Sensor darüber Aufschluß gibt. ob die Belastung echt oder nur scheinbar ist.

Ein Gerät der eingangs genannten Art ist aus der EP-A-0 080 348 bekannt. Es handelt sich dabei um einen implantierbaren Herzschrittmacher, in dessen Gehäuse ein piezoelektrischer Drucksensor eingebaut ist, der während körperlicher Aktivitäten des den Herzschrittmacher tragenden Lebewesens durch die Bewegungen der Muskeln und dergleichen entstehende mechanische Schwingungen im Körper des Lebewesens, die sich als Druckwellen ausbreiten, aufnimmt und in ein entsprechendes elektrisches Signal umwandelt. Anhand dieses Signals wird die Stimulationsintensität, d.h. die Stimulationsfrequenz, mit der der Herzschrittmacher das Herz beim Ausbleiben von natürlichen Herzschlägen stimuliert, in Abhängigkeit von der körperlichen Aktivität des Lebewesens eingestellt. Dabei besteht die Möglichkeit, die Empfindlichkeit. mit der die Anpassung der Stimulationsfrequenz anhand des Signales des piezoelektrischen Drucksensors erfolgt, ausgehend von einem Maximalwert zu verringern, um eine Anpassung an die individuellen Gegebenheiten zu ermöglichen. Die Anpassung der Stimulationsfrequenz erfolgt zwischen einem oberen und einem unteren Grenzwert. wobei der obere Grenzwert bei Zuständen sehr hoher körperlicher Aktivität erreicht wird. während der untere Grenzwert im Ruhezustand, z.B. während des Schlafens. wirksam ist. Insbesondere während des Schlafens tritt das Problem auf, dass das den Herzschrittmacher tragende Lebewesen eine solche Position einnimmt, dass auf den piezoelektrischen Drucksensor eine Druckausübung erfolgt. Dieser erzeugt dann ein Signal, ohne dass eine körperliche Aktivität des Lebewesens vorliegt. Dies führt zu einer Anhebung der Stimulationsfrequenz. was für das Lebewesen, das sich im Zustand des Schlafs befindet, äusserst unangenehm ist.

Der Erfindung liegt die Aufgabe zugrunde, ein Gerät der eingangs genannten Art so auszubilden, dass während Phasen, in denen sich der Körper des das Gerät tragenden Lebewesens im Zustand der Ruhe, insbesondere im Zustand des Schlafs, befindet, die Gefahr von unbegründeten Steigerungen der Stimulationsintensität vermindert ist.

Der Aufgabe wird nach der Erfindung dadurch gelöst, dass das Gerät ausserdem Mittel zum Ermitteln der Stimulationsintensität. Mittel zum Vergleichen der Stimulationsintensität. die die jeweils ermittelte Stimulationsintensität mit einem Schwellwert vergleichen, Mittel zum Ermitteln einer Zeitdauer, die die Zeitdauer ermitteln, für die die Stimulationsintensität den Schwellwert unterschreitet, und Mittel zum Vergleichen von Zeitdauern, die die ermittelte Zeitdauer mit einer Mindestzeitdauer vergleichen. aufweist. wobei bei einem Überschreiten der Mindestzeitdauer eine Absenkung der Empfindlichkeit auf einen unterhalb des Normalwertes liegenden Empfindlichkeitswert erfolgt und, daß die Sensoreinrichtung einen einzigen Sensor aufweist, der ein mit dem Körper des Lebewesens mechanisch in Kontakt stehender piezoelektrischer Drucksensor ist. Ist also die Stimulationsintensität über einen gewissen Mindestzeitraum, z.B. 30 Minuten, hinweg auf einen Wert abgesunken, der z.B. wenig oberhalb der im Ruhezustand bzw. im Zustand des Schlafes vorliegenden Stimulationsintensität liegt, wird davon ausgegangen, dass sich das das Gerät tragende Lebewesen tatsächlich im Zustand der Ruhe bzw. im Zustand des Schlafes befindet, und eine Absenkung der Empfindlichkeit auf einen unterhalb des Normalwertes liegenden Empfindlichkeitswert vorgenommen. Damit ist sichergestellt, dass von der Sensoreinrichtung stammende Signale im Gegensatz zum Stand der Technik nicht zu einer unerwünschten Anhebung der Stimulationsintensität führen können.

Gemäss einer Variante der Erfindung ist vorgesehen, dass die Mittel zum Vergleichen der Stimulationsintensität die jeweils vorliegende Stimulationsintensität ausserdem mit einem zweiten Schwellwert vergleichen. der unterhalb des Schwellwertes liegt, dass die Mittel zum Ermitteln einer Zeitdauer ausserdem eine zweite Zeitdauer ermitteln, für die die Stimulationsintensität den zweiten Schwellwert unterschreitet, und dass die Mittel zum Vergleichen von Zeitdauern ausserdem die ermittelte zweite Zeitdauer mit einer zweiten Mindestzeitdauer vergleichen, wobei bei einem Überschreiten der zweiten Mindestzeitdauer eine Absenkung der Empfindlichkeit der Stellmittel auf einen unterhalb des Empfindlichkeitswertes liegenden zweiten Empfindlichkeitswert erfolgt. Durch diese Massnahme besteht in vorteilhafter Weise die Möglichkeit, im Anschluss an eine erste sozusagen nur versuchsweise vorgenommene Absenkung der Empfindlichkeit eine weitere Absenkung der Empfindlichkeit auf einen niedrigeren zweiten Empfindlichkeitswert vorzunehmen. nachdem sichergestellt ist. dass sich das Lebewesen tatsächlich in einem länger andauernden Zustand der Ruhe oder des Schlafes befindet und seine körperliche Aktivität nicht wieder erhöht hat.

Um eine gleichmässige Arbeitsweise des Gerätes zu gewährleisten ist es zweckmässig, Mittel zur Bildung eines Mittelwertes vorzusehen. die den zeitlichen Mittelwert der ermittelten Stimulationsintensität bilden. wobei die Mittel zum Vergleicnen der Stimulationsintensität den Mittelwert der Stimulationsintensität mit einem Schwellwert für den Mittelwert der Stimulationsintensität vergleichen und die Mittel zum Ermitteln einer Zeitdauer diejenige Zeitdauer ermitteln, für die der zeitliche Mittelwert der Stimulationsintensität den Schwellwert für den Mittelwert der Stimulationsintensität unterschreitet

Gemäss einer. Ausführungsform der Erfindung ist vorgesehen, dass das Gerät als Herzschrittmacher ausgebildet ist, wobei die Mittel zum Stimulieren die Herztätigkeit des Lebewesens stimulieren und die Stellmittel zur Anpassung der Stimulationsintensität die Stimulationsfrequenz an die körperliche Aktivität des Lebewesens anpassen.

Ein Ausführungsbeispiel der Erfindung ist anhand der beigefügten Zeichnungen erläutert. Es zeigen:
- FIG 1: ein Blockschaltbild eines erfindungsgemäss ausgebildeten Herzschrittmachers. und
- FIG 2: ein die Funktion des Gerätes nach FIG 1 verdeutlichendes Schema.

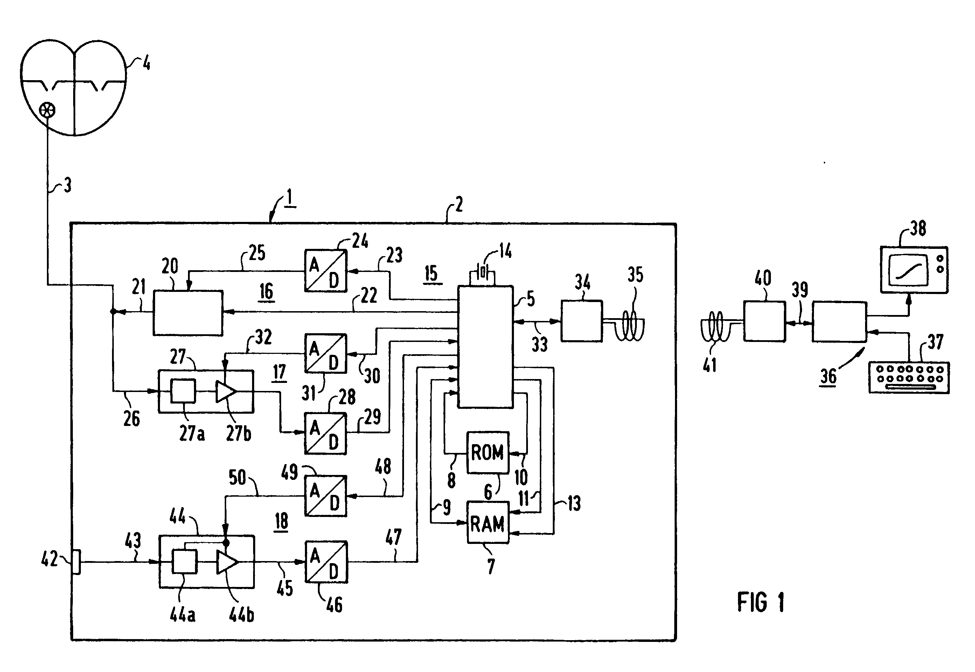

In der FIG 1 ist die Erfindung anhand eines insgesamt mit (1) bezeichneten Herzschrittmachers dargestellt. Die Bauteile des Herzschrittmachers (1) sind in einem schematisch angedeuteten hermetisch dichten Gehäuse (2) aufgenommen, das zur Implantation in den Körper eines Lebewesens geeignet ist. Von dem im VVI-Mode arbeitenden Herzschrittmacher (1) führt eine Elektrode (3) zu einem Herz (4) eines Lebewesens und ist dort in ein Ventrikel, vorzugsweise das durch das Venensystem zugängliche rechte Ventrikel, eingepflanzt.

Der Herzschrittmacher (1) umfasst unter anderem einen Mikroprozessor (5), dem ein Nur-Lese-Speicher (ROM) (6) und ein Schreib-Lese-Speicher (RAM) (7) zugeordnet sind. die über Datenleitungen (8.9) und Adressleitungen (10.11) mit dem Mikroprozessor (5) in Verbindung stehen. Zu dem RAM (7) führt von dem Mikroprozessor (5) ausserdem eine Leitung (13), die zum Umschalten des RAM (7) von Schreib- auf Lesebetrieb und umgekehrt dient. In dem ROM (6) ist ein Programm gespeichert. mittels dessen sämtliche Funktionen des Herzschrittmachers (1) gesteuert werden. Wenn also im folgenden die Rede davon ist. dass der Mikroprozessor (5) eine bestimmte Funktion ausführt. so ist darunter zu verstehen, dass der Mikroprozessor (5) unter Ausführung des im ROM (6) gespeicherten Programmes bei Heranziehung von im RAM (7) befindlichen Daten und ihm anderweitig, z.B. über eine Eingangs-Beschaltung, zugeführter Daten zur Ausführung der jeweiligen Funktion tätig wird.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16,17,18) aufweist.

Der Kanal (16) dient dazu, das Herz (4) erforderlichenfalls mit Stimulationsimpulsen zu versorgen. Der Kanal (16) besitzt daher einen Stimulationsimpuls-Generator (20), dessen Ausgangsleitung (21) mit der Elektrode (3) verbunden ist. Der Stimulationsimpuls-Generator (20) ist über eine Leitung (22), die mit einem entsprechenden Ausgang des Mikroprozessors (5) verbunden ist, zur Abgabe eines elektrischen Stimulationsimpulses aktivierbar. Digitale Daten, die die Form der Stimulationsimpulse, z.B. deren Amplitude und Dauer, betreffen, gelangen von dem Mikroprozessor (5) über eine Leitung (23) zu einer Digital/Analog-Schnittstelle (24), die dem Stimulationsimpuls-Generator (20) über eine Steuerleitung (25) den digitalen Daten entsprechende analoge Steuersignale zuführt, die den Stimulationsimpuls-Generator (20) derart einstellen, dass er bei Bedarf Stimulationsimpulse der gewünschten Form erzeugt.

Der Kanal (17) besitzt eine über eine Eingangsleitung (26) ebenfalls mit der Elektrode (3) verbundene Signalaufbereitungsschaltung (27), die dazu dient, ein mittels der Elektrode (3) vom Herzen (4) abgenommenes, der Aktivität des Herzens entsprechendes elektrisches Signal zu filtern und zu verstärken. Die Signalaufbereitungsschaltung (27) umfasst daher ein Filter (27a) und einen Verstärker (27b). Vom Ausgang der Signalaufbereitungsschaltung (27) gelangt das aufbereitete Signal zu einem Analog/Digital-Wandler (28). Von diesem gelangen über eine Leitung (29) digitale Daten zu einem entsprechenden Eingang des Mikroprozessors (5), die dem Verlauf des am Ausgang der Signalaufbereitungsschaltung (27) vorliegenden elektrischen Signales entsprechen, das seinerseits die elektrische Aktivität des Herzens (4) widerspiegelt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die Signalaufbereitungsschaltung (27) weitergibt. Die digitalen Daten bzw. die entsprechenden analogen Signale dienen dazu, z.B. den Verstärkungsfaktor des Verstärkers (27b) einzustellen bzw. den Verstärker (27b) vollständig zu sperren.

Die über die Leitung (29) dem Mikroprozessor (5) zugeführten digitalen Daten analysiert dieser daraufhin, ob in dem der Aktivität des Herzens (4) entsprechenden elektrischen Signal Ereignisse enthalten sind, die dem Auftreten eines natürlichen Herzschlages entsprechen. Detektiert der Mikroprozessor (5) einen natürlichen Herzschlag oder aktiviert er über die Leitung (22) den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulsen abzuzählen, die einem zwischen einer oberen und einer unteren Grenze einstellbaren Zeitintervall entspricht. Das jeweils eingestellte Zeitintervall bestimmt diejenige Stimulationsfrequenz, mit der das Herz (4) beim Ausbleiben natürlicher Herzschläge stimuliert wird. Gelangen während dieses Zeitintervalles über den Kanal (17) keine Daten zu dem Mikroprozessor (5), die dieser als natürlichen Herzschlag detektiert, aktiviert der Mikroprozessor (5) bei Ablauf des Zeitintervalls über die Leitung (22) den Stimulationsimpuls-Generator (20). Im Anschluss an die Abgabe eines Stimulationsimpulses beginnt der Mikroprozessor (5) erneut eine Anzahl von Taktimpulsen abzuzählen, die dem jeweils eingestellten, die Stimulationsfrequenz bestimmenden Zeitintervall entspricht. Detektiert der Mikroprozessor (5) dagegen während des Laufs des jeweils eingestellten, die Stimulationsfrequenz bestimmenden Zeitintervalls einen natürlichen Herzschlag, bricht er den beschriebenen Zählvorgang ab, sofern ein weiteres Zeitintervall, die sogenannte Refraktärzeit, abgelaufen ist, und beginnt den beschriebenen Zählvorgang von neuem.

Die Refraktärzeit, die grundsätzlich kürzer ist als das zwischen beispielsweise 400 und 2000 ms einstellbare, die Stimulationsfrequenz bestimmende Zeitintervall, dauert zwischen etwa 250 und 450 ms (einstellbar). Die Refraktärzeit unterteilt sich in eine absolute Refraktärzeit mit einer festen Dauer von gewöhnlich 125 ms und eine relative Refraktärzeit, auf die der restliche Teil der gesamten jeweils eingestellten Refraktärzeit entfällt. Die Refraktärzeit beginnt jeweils gleichzeitig mit dem die Stimulationsfrequenz bestimmenden Zeitintervall zu laufen und wird von dem Mikroprozessor (5) im Zuge des gleichen Zählvorgangs ermittelt, der auch zur Ermittlung des die Stimulationsfrequenz bestimmenden Zeitintervalles dient. Während der absoluten Refraktärzeit ist im Kanal (17) der Verstärker (27b) der Signalaufbereitungsschaltung (27) vollständig gesperrt, was dadurch erreicht wird, dass der Mikroprozessor (5) den Verstärker (27b) über die Leitung (30), die Digital/Analog-Schnittstelle (31) und die Steuerleitung (32) mit einem entsprechenden Steuersignal beaufschlagt. Infolge der vollständigen Sperrung des Verstärkers (27b) ist mittels des Mikroprozessors (5) während der Dauer der absoluten Refraktärzeit keinerlei Detektion möglich. Nach Ablauf der absoluten Refraktärzeit aktiviert der Mikroprozessor (5) den Verstärker (27b) wieder, so dass er in der Lage ist, natürliche Herzschläge zu detektieren. Detektiert der Mikroprozessor (5) während der relativen Refraktärzeit einen natürlichen Herzschlag, bricht er im Gegensatz zu einer Detektion nach Ablauf der Refraktärzeit den Zählvorgang zur Ermittlung des jeweils eingestellten, die Stimulationsfrequenz bestimmenden Zeitintervalles nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils eingestellte Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils eingestellte Zeitintervall bestimmten Stimulationsfrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Mikroprozessor (5) ist über eine Leitung (33) mit einem Telemetrieschaltkreis (34) verbunden, an den eine Sende/Empfangs-Spule (35) angeschlossen ist. Der Herzschrittmacher (1) ist somit in der Lage, mit einem externen Programmiergerät (36) mit einer Tastatur (37) und einem Monitor (38) Daten auszutauschen, da das Programmiergerät (36) über eine Leitung (39) mit einem zweiten Telemetrieschaltkreis (40) verbunden ist, an den wieder eine Sende/Empfangs-Spule (41) angeschlossen ist. Zum Datenaustausch zwischen dem Herzschrittmacher (1) und dem Programmiergerät (36) wird die Sende/Empfangs-Spule (41) des zu dem Programmiergeräts (36) gehörigen Telemetrieschaltkreises (40) so auf der Körperoberfläche des den Herzschrittmacher (1) tragenden Lebewesens positioniert, dass sie mit der Sende-Empfangs-Spule (35) des Herzschrittmachers (1) induktiv gekoppelt ist. Es besteht dann die Möglichkeit, dem Programmiergerät (36) die in dem ROM (6) und dem RAM (7) befindlichen Daten zur Überprüfung bzw. Überprüfung und Veränderung zuzuführen. Ausserdem besteht die Möglichkeit, dem RAM (7) des Herzschrittmachers (1) von dem Programmiergerät (36) her veränderte bzw. zusätzliche Daten zuzuführen.

Der Kanal (18) der Eingangs/Ausgangs-Schaltung (15) des Mikroprozessors (5) dient dazu, dem Mikroprozessor (5) Daten zur Verfügung zu stellen, die es diesem anhand des in dem ROM (6) gespeicherten Programmes gestatten, die Stimulationsintensität, also das der gewünschten Herzschlagfrequenz entsprechende Zeitintervall, an die körperliche Aktivität des den Herzschrittmacher (1) tragenden Lebewesens anzupassen. Zu diesem Zweck ist ein piezoelektrischer Druck-Sensor (42) vorgesehen, der mit der Wandung des Gehäuses (2) verbunden ist und somit mittelbar mit dem Körper des Lebewesens in mechanischem Kontakt steht. Während körperlicher Aktivitäten des Lebewesens entstehen durch die Bewegung der Muskeln und dergleichen mechanische Schwingungen im Körper des Lebewesens, die sich als Druckwellen in dem Körper des Lebewesens ausbreiten und von dem piezoelektrischen Sensor (42) aufgenommen und in elektrische Signale umgewandelt werden. Diese Signale, deren Amplitude mit zunehmender körperlicher Aktivität ebenfalls zunimmt, gelangen über eine Leitung (43) zu einer Signalaufbereitungsschaltung (44), die ein Filter (44a) mit nachfolgendem Verstärker (44b) enthält. Das Ausgangssignal der Signalaufbereitungsschaltung (44) gelangt über eine Leitung (45) zu einem Analog/Digital-Wandler (46), z.B. einem 8-bit-Wandler, dessen digitale Ausgangssignale über eine Leitung (47) zu dem Mikroprozessor (5) gelangen. Der Mikroprozessor (5) ist über eine Leitung (48) mit einer Digital/Analog-Schnittstelle (49) verbunden, die ihr von dem Mikroprozessor (5) zugeführte digitale Daten als entsprechende analoge Signale über eine Steuerleitung (50) an die Signalaufbereitungsschaltung (44) weitergibt. Die digitalen Daten bzw. die diesen entsprechenden analogen Signale dienen dazu, z.B. den Verstärkungsfaktor des Verstärkers (44b) einzustellen oder die Charakteristik des Filters (44a) zu verändern. In Abhängigkeit von dem zeitlichen Verlauf des von dem piezoelektrischen Sensor (42) stammenden Signals bzw, der entsprechenden digitalen Daten verändert der Mikroprozessor (5) in ähnlicher Weise, wie dies in der EP-A-0 080 348 beschrieben ist, das die Stimulationsfrequenz bestimmende Zeitintervall derart, dass dieses mit zunehmender körperlicher Aktivität verkürzt wird. Dies vollzieht sich zwischen einer unteren Grenze (Ruhepuls) und einer oberen Grenze (maximale Herzschlagfrequenz), die den Bedürfnissen des jeweiligen Lebewesens entsprechend gewählt werden. Entsprechende Daten werden telemetrisch in das RAM (7) eingegeben.

Wie bereits angedeutet wurde, besteht die Gefahr, dass das Lebewesen, während es sich körperlich im Zustand der Ruhe, z.B. im Zustand des Schlafes, befindet, eine solche Position einnimmt, dass infolge seines Körpergewichtes eine Druckausübung auf den piezoelektrischen Drucksensor (42) stattfindet, mit der Folge, dass das der gewünschten Herzschlagfrequenz entsprechende Zeitintervall verkürzt wird, obwohl dies nicht erforderlich ist. Um hier Abhilfe zu schaffen, ist das in dem ROM (6) gespeicherte Programm so beschaffen, dass der Mikroprozessor (5), evtl. unter Heranziehung von in dem RAM (7) gespeicherten Daten, fortlaufend eine der tatsächliche vorliegenden Herzschlagfrequenz entsprechende Grösse F ermittelt. Die der Herzschlagfrequenz entsprechende Grösse F vergleicht der Mikroprozessor mit einem entsprechenden Schwellwert SWF 1. Er ermittelt ausserdem diejenige Zeitdauer T 1, für die, die der Herzschlagfrequenz entsprechende Grösse F den Schwellwert SWF 1 unterschreitet. Die Zeitdauer T 1 vergleicht der Mikroprozessor (5) mit einer Mindestzeitdauer SWT 1. Stellt der Mikroprozessor (5) fest, dass die Mindestzeitdauer SWT 1 überschritten wurde, nimmt er eine Absenkung der Empfindlichkeit E der Signalaufbereitungsschaltung (44) vor, d.h., er senkt den Verstärkungsfaktor des Verstärkers (44b) derart ab, dass anstelle eines zunächst vorhandenen Normalwertes N der Empfindlichkeit E ein niedrigerer Empfindlichkeitswert E 1 vorliegt. Dabei erfolgt die Absenkung der Empfindlichkeit E bzw. die Verringerung des Verstärkungsfaktors, indem der Mikroprozessor (5) der Digital/Analog-Schnittstelle (49) entsprechende digitale Daten zuführt, die diese in ein geeignetes Steuersignal wandelt, das über die Steuerleitung (50) zu der Signalaufbereitungsschaltung (44) bzw. deren Verstärker (44b) gelangt. Der Empfindlichkeitswert E 1 ist dabei so gewählt, dass nur Druckeinwirkungen auf den piezoelektrischen Drucksensor (42), deren Stärke einer merklich erhöhten körperlichen Aktivität des Lebewesens entspricht, den Mikroprozessor (5) zu einer Verkürzung des der gewünschten Herzschlagfrequenz entsprechenden Zeitintervalles veranlassen können.

Tritt dies während einer zweiten Mindestzeitdauer SWT 2 nicht ein und liegt während dieser zweiten Mindestzeitdauer SWT 2 die der Herzschlagfrequenz entsprechenden Grösse F unterhalb eines zweiten Schwellwertes SWF 2, der kleiner oder gleich dem Schwellwert SWF 1 ist, erfolgt eine nochmalige Absenkung der Empfindlichkeit E auf einen unterhalb des Empfindlichkeitswertes E 1 liegenden zweiten Empfindlichkeitswert E 2. Die Arbeitsweise des Gerätes entspricht dabei der im Zusammenhang mit der ersten Absenkung der Empfindlichkeit E beschriebenen.

Verharrt das den Herzschrittmacher tragende Lebewesen also auch während der Mindestzeitdauer SWT 2 im Zustand der Ruhe bzw. im Zustand des Schlafes, erfolgt eine nochmalige Absenkung der Empfindlichkeit E. Dabei ist der zweite Empfindlichkeitswert E 2 so gewählt, dass eine Verkürzung des der gewünschten Herzschlagfrequenz entsprechenden Zeitintervalles tatsächlich nur dann auftreten kann, wenn das Lebewesen eine körperliche Aktivität erheblicher Intensität aufnimmt.

Ist die Empfindlichkeit E des Verstärkers (44b) auf den Empfindlichkeitswert E 1 bzw. auf den Empfindlichkeitswert E 2 abgesenkt und ergibt der Vergleich der der Herzschlagfrequenz entsprechenden Grösse F mit dem Schwellwert SWF 1 bzw. dem Schwellwert SWF 2, dass der jeweilige Schwellwert überschritten wird, wird die Empfindlichkeit E auf ihren Normalwert N bzw. den Empfindlichkeitswert E 1 angehoben.

Um zu vermeiden, dass bereits geringfügige und kurzfristige Schwankungen der Herzschlagfrequenz des Lebewesens zu Veränderungen der Empfindlichkeit E führen können, ermittelt der Mikroprozessor (5) als der Herzschlagfrequenz entsprechende Grösse F den zeitlichen Mittelwert der Herzschlagfrequenz über eine Anzahl z von Gerätezyklen, die unter Berücksichtigung der Schwellwerte SWF 1 und SWF 2 so gewählt ist, dass die Mittelwertbildung über einen Zeitraum erfolgt, der wenigstens der grösseren der beiden Mindestzeitdauern SWT 1 bzw. SWT 2 entspricht. Zweckmässigerweise erfolgt die Ermittlung des Mittelwertes dadurch, dass die während der Anzahl von z Zyklen jeweils eingestellten der gewünschten Herzschlagfrequenz entsprechenden Zeitintervalle aufaddiert und die Summe durch die Anzahl z der Zyklen dividiert wird.

Grundsätzlich besteht aber auch die Möglichkeit, die tatsächliche Herzschlagfrequenz anhand der abgegebenen Stimulationsimpulse und der detektierten natürlichen Herzschläge sowie der zwischen diesen Ereignissen verstrichenen Zeitintervalle zu ermitteln.

Die Zeitdauern T 1 und T 2 für die die Schwellwerte SWF 1 und SWF 2 unterschritten werden, ermittelt der Mikroprozessor (5) durch Addition der Dauern der seit dem Unterschreiten des jeweiligen Schwellwertes SWF 1 bzw. SWF 2 liegenden Gerätezyklen.

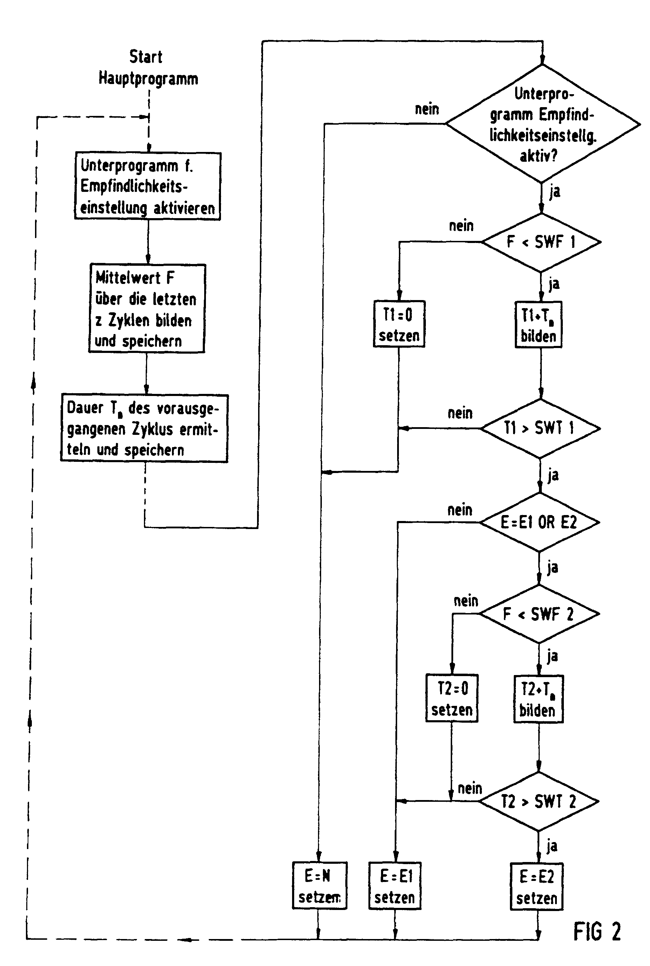

Der zuvor beschriebene Sachverhalt wird anhand der FIG 2 nochmals deutlicher, die in schematischer Darstellung die Programmschleife des in dem ROM (6) gespeicherten Programmes zeigt, soweit dies für das Verständnis der vorliegenden Erfindung wesentlich ist. Dieses Programm besteht aus einem Hauptprogramm und einer Reihe von Unterprogrammen, von denen in FIG 2 dasjenige dargestellt ist, mittels dessen die Empfindlichkeit E für die Signale des piezoelektrischen Drucksensors (42) in der zuvor beschriebenen Weise eingestellt wird. Von dem Hauptprogramm, das im wesentlichen als strichliert angedeutete Schleife dargestellt ist, sind nur einige wenige Operationen aufgeführt, die zur Durchführung des dargestellten Unterprogrammes erforderlich sind.

Gemäss FIG 2 besteht im Rahmen des Hauptprogrammes, das, nachdem es gestartet wurde, pro Gerätezyklus einmal in Form einer Programmschleife abgearbeitet wird, zunächst die Möglichkeit, das Unterprogramm zur Empfindlichkeitseinstellung zu aktivieren. Nur wenn dies der Fall ist, werden die erfindungsgemässen Funktionen ausgeführt. Weiter wird im Rahmen des Hauptprogrammes der Mittelwert der Herzschlagfrequenz für die letzten z Gerätezyklen gebildet und gespeichert sowie die Dauer des vorausgegangenen Gerätezyklus ermittelt und gespeichert. Diese Operationen, die an sich auch Bestandteil des Unterprogrammes zur Empfindlichkeitseinstellung sein könnten, werden im Rahmen des Hauptprogrammes ausgeführt, da die ermittelten Daten auch für andere Zwecke als die der Empfindlichkeitseinstellung brauchbar sind. An geeigneter Stelle in dem Hauptprogramm wird überprüft, ob das Unterprogramm zur Empfindlichkeitsstellung aktiv ist. Ist dies der Fall wird im Rahmen des Unterprogrammes zunächst geprüft, ob der aktuelle Mittelwert F der Herzschlagfrequenz den Schwellwert SWF 1 unterschreitet. Ist dies der Fall wird der Inhalt eines Registers, in dem die seit dem Unterschreiten des Schwellwertes SWF 1 verstrichene Zeit T 1 gespeichert ist, um die Dauer Tₙ des vorausgegangenen Gerätezyklus vermehrt. Ergibt der Vergleich der so erhaltenen aktuellen Zeitdauer T 1 mit der Mindestzeitdauer SWT 1 dass diese überschritten ist, wird als nächstes überprüft, ob die im Moment eingestellte Empfindlichkeit E einen der Empfindlichkeitswerte E 1 oder E 2 besitzt. Ist dies der Fall, wird geprüft, ob der aktuelle Mittelwert F der Herzschlagfrequenz den zweiten Schwellwert SWF 2 ebenfalls unterschreitet. Ist dies der Fall, wird in der zuvor im Zusammenhang mit der Zeitdauer T 1 beschriebenen Weise auch die Zeitdauer T 2 ermittelt. Diese wird dann mit der Mindestzeitdauer SWT 2 verglichen. Überschreitet sie diese, wird die Empfindlichkeit E, unabhängig davon, ob dies zuvor bereits der Fall war oder nicht, auf den Wert E 2 gesetzt, worauf die Rückkehr ins Hauptprogramm erfolgt.

Für den Fall, dass das Unterprogramm zur Empfindlichkeitseinstellung nicht aktiviert ist, wird vor der Rückkehr ins Hauptprogramm die Empfindlichkeit E auf den Normalwert N eingestellt. Hierdurch wird sichergestellt, dass nach einer Deaktivierung des Unterprogramms etwaige von dem Normalwert N abweichende Einstellungen der Empfindlichkeit E rückgängig gemacht werden. Ergibt die Überprüfung bezüglich des Schwellwertes SWF 1, dass dieser nicht unterschritten wird, werden die Zeitdauer T 1 auf Null und die Empfindlichkeit E auf ihren Normalwert N eingestellt. Dies ist erforderlich, um sicherzustellen, dass nach einem vorangegangenen Unterschreiten des Schwellwertes SWF 1 die in diesem Zusammenhang ermittelte Zeitdauer T 1 gelöscht und eine nach einem evtl. vorangegangenen Überschreiten der Mindestzeitdauer SWT 1 erfolge Absenkung der Empfindlichkeit E wieder aufgehoben wird. Ergibt die Überprüfung bezüglich der Mindestzeitdauer SWT 1 dass die Zeitdauer T 1 diese noch nicht übersteigt, erfolgt die Rückkehr ins Hauptprogramm nachdem die Empfindlichkeit E auf ihren Normalwert N eingestellt wurde. In entsprechender Weise wird für den Fall, dass keine Unterschreitung des Schwellwertes SWF 2 vorliegt die Zeitdauer T 2 auf Null und die Empfindlichkeit E auf den Empfindlichkeitswert E 1 eingestellt, während für den Fall, dass eine Überschreitung der Mindestzeitdauer SWT 2 nicht vorliegt lediglich die Empfindlichkeit E auf den Empfindlichkeitswert E 1 eingestellt wird, bevor die Rückkehr ins Hauptprogramm erfolgt.

Die beschriebenen Vorgänge wiederholen sich im Falle des beschriebenen Ausführungsbeispiels für jeden Gerätezyklus. Es besteht jedoch auch die Möglichkeit, nur den Mittelwert F der Herzschlagfrequenz und die Dauern T der Gerätezyklen fortlaufend zu ermitteln und die Überprüfung der Empfindlichkeitseinstellung nur jeweils nach Ablauf eines definierten Zeitintervalles vorzunehmen.

Anders als bei dem beschriebenen Ausführungsbeispiel, wo die Einstellung der Empfindlichkeit durch Beeinflussung des Verstärkungsfaktors des Verstärkers (44b) der dem piezoelektrischen Drucksensor (42) zugeordneten Signalaufbereitungsschaltung (44) erfolgt, besteht grundsätzlich auch die Möglichkeit eine Einstellung der Empfindlichkeit dadurch vorzunehmen, dass die dem Mikroprozessor (5) von dem Digital/Analog-Wandler (46) mit einem geeigneten Faktor multipliziert werden, bevor sie weiterverarbeitet werden. In diesem Falle erfolgt die Einstellung der Empfindlichkeit also auf rechnerischem Wege.

Im Falle des Ausführungsbeispieles sind eine Reihe von Funktionen durch einen entsprechend programmierten Mikroprozessor (5) realisiert. Es besteht jedoch auch die Möglichkeit, entsprechende elektrische Schaltungen vorzusehen, die diese Funktionen realisieren.

Nimmt man an, dass der Normalwert N der Empfindlichkeit E 100 % entspricht, sollten der Empfindlichkeitswert E 1 im Bereich von 70 % und der Empfindlichkeitswert E 2 in der Grössenordnung von 40 % liegen. Günstige Werte für die Mindestzeitdauern SWT 1 und SWT 2 sind 10 min bzw. 30 min. Die Schwellwerte SWF 1 und SWF 2 für den zeitlichen Mittelwert F der Herzschlagfrequenz sind vom jeweiligen Patienten abhängig. Als Richtwerte können SWF 1 = 1,33 Schläge pro Sekunde und SWF 2 = 1,17 Schläge pro Sekunde herangezogen werden.

Die Erfindung wurde ausschliesslich anhand eines Herzschrittmachers erläutert. Sie kann jedoch auch bei anderen Geräten zur Stimulation physiologischer Vorgänge Verwendung finden.

## Patentansprüche

1. In den Körper eines Lebewesens implantierbares medizinisches Gerät mit Mitteln (3,5,20) zum Stimulieren eines physiologischen Vorgangs des Lebewesens mit einstellbarer Stimulationsintensität und mit selbsttätigen Stellmitteln (5,24) zur Anpassung der Stimulationsintensität an die körperliche Aktivität des Lebewesens, die die Stimulationsintensität anhand eines der körperlichen Aktivität des Lebewesens entsprechenden Signals einer Sensoreinrichtung (42,44,46) einstellen, wobei die Empfindlichkeit (E), mit der die Einstellung der Stimulationsintensität anhand des Signals der Sensoreinrichtung (42,44,46) erfolgt, ausgehend von einem Normalwert (N) verringerbar ist, **dadurch gekennzeichnet, dass** das Gerät ausserdem Mittel (5,6,7) zum Ermitteln einer der Stimulationsintensität entsprechenden Grösse (F), Mittel (5,6,7) zum Vergleichen der Stimulationsintensität, die die jeweils ermittelte der Stimulationsintensität entsprechende Grösse (F) mit einem entsprechenden Schwellwert (SWF 1) vergleichen, Mittel (5,6,7,14) zum Ermitteln einer Zeitdauer, die die Zeitdauer (T 1) ermitteln, für die die der Stimulationsintensität entsprechende Grösse (F) den Schwellwert (SWF 1) unterschreitet, und Mittel (5.6.7) zum Vergleichen von Zeitdauern, die die ermittelte Zeitdauer (T 1) mit einer Mindestzeitdauer (SWT 1) vergleichen, aufweist, wobei bei einem Überschreiten der Mindestzeitdauer (SWT 1) eine Absenkung der Empfindlichkeit (E) auf einen unterhalb des Normalwertes (N) liegenden Empfindlichkeitswert (E 1) erfolgt und, daß die Sensoreinrichtung einen einzigen Sensor aufweist, der ein mit dem Körper des Lebewesens mechanisch in Kontakt stehender piezoelektrischer Drucksensor (42) ist.

2. Gerät nach Anspruch 1, **dadurch gekennzeichnet, dass** die Mittel (5.6.7) zum Vergleichen der Stimulationsintensität die ermittelte der Stimulationsintensität entsprechende Grösse (F) mit einem zweiten Schwellwert (SWF 2) vergleichen, der gleich oder kleiner als der Schwellwert (SWF 1) ist. dass die Mittel (5.6.7.14) zum Ermitteln einer Zeitdauer ausserdem eine zweite Zeitdauer (T 2) ermitteln. für die die der Stimulationsintensität entsprechende Grösse (F) den zweiten Schwellwert (SWF 2) unterschreitet, und dass die Mittel (5,6,7) zum Vergleichen von Zeitdauern ausserdem die ermittelte zweite Zeitdauer (T 2) mit einer zweiten Mindestzeitdauer (SWT 2) vergleichen. wobei bei einem Überschreiten der zweiten Mindestzeitdauer (SWT 2) eine Absenkung der Empfindlichkeit (E) auf einen unterhalb des Empfindlichkeitswertes (E 1) liegenden zweiten Empfindlichkeitswert (E 2) erfolgt.

3. Gerät nach Anspruch 1 oder Anspruch 2. **dadurch gekennzeichnet, dass** die Mittel (5,6,7) zur Bestimmung einer der Stimulationsintensität entsprechenden Grösse (F) den zeitlichen Mittelwert der Stimulationsintensität bilden. wobei die Mittel (5,6,7) zum Vergleichen der Stimulationsintensität den Mittelwert der Stimulationsintensität mit einem Schwellwert (SWF 1. SWF 2) für den Mittelwert der Stimulationsintensität vergleichen und die Mittel (5,6,7,14) zum Ermitteln einer Zeitdauer diejenige Zeitdauer (T 1. T 2) ermitteln, für die der zeltliche Mittelwert der Stimulationsintensität den Schwellwert (SWF 1. SWF 2) für den Mittelwert der Stimulationsintensität unterschreitet.

4. Gerät nach einem der Ansprüche 1 bis 3, **dadurch gekennzeichnet, dass** es als Herzschrittmacher ausgebildet ist. wobei die Mittel (3,5.20) zum Stimulieren die Herztätigkeit des Lebewesens stimulieren und die Stellmittel (5.24) zur Anpassung der Stimulationsintensität die Stimulationsfrequenz an die körperliche Aktivität des Lebewesens anpassen.

## Claims

1. Medical apparatus which is implantable into the body of a living being and which has means (3, 5, 20) for stimulating a physiological process of the living being with settable stimulation intensity and having automatic actuating means (5, 24) for adapting the stimulation intensity to the body activity of the living being, which actuating means set the stimulation intensity with reference to a signal of a sensor device (42, 44, 46), which signal corresponds to the body activity of the living being, in which the sensitivity (E) with which the setting of the stimulation intensity with reference to the signal of the sensor device (42, 44, 46) takes place can be reduced, proceeding from a normal value (N), **characterized in that** the apparatus further exhibits means (5, 6, 7) for determining a magnitude (F) corresponding to the stimulation intensity, means (5, 6, 7) for comparing the stimulation intensity, which comparing means compare the respectively determined magnitude (F) corresponding to the stimulation intensity with a corresponding threshold value (SWF 1), means (5, 6, 7, 14) for determining a time duration, which means for determining a time duration determine the time duration (T1) for which the magnitude (F) corresponding to the stimulation intensity falls below the threshold value (SWF 1), and means (5, 6, 7) for comparing time durations, which means for comparing time durations compare the determined time duration (T 1) with a minimum time duration (SWT 1), in which, if the minimum time duration (SWT 1) is exceeded, a lowering of the sensitivity (E) to a sensitivity value (E 1) lying below the normal value (N) takes place, and **in that** the sensor device comprises a single sensor, which is a piezoelectric pressure sensor (42) that is mechanically in contact with the body of the living being.

2. Apparatus according to Claim 1, **characterized in that** the means (5, 6, 7) for comparing the stimulation intensity compare the determined magnitude (F) corresponding to the stimulation intensity with a second threshold value (SWF 2), which is equal to or smaller than the threshold value (SWF 1), **in that** the means (5, 6, 7, 14) for determining a time duration further determine a second time duration (T 2) for which the magnitude (F) corresponding to the stimulation intensity falls below the second threshold value (SWF 2), and **in that** the means (5, 6, 7) for comparing time durations further compare the determined second time duration (T 2) with a second minimum time duration (SWT 2), in which, if the second minimum time duration (SWT 2) is exceeded, a lowering of the sensitivity (E) to a second sensitivity value (E 2) lying below the sensitivity value (E 1) takes place.

3. Apparatus according to claim 1 or claim 2, **characterized in that** the means (5, 6, 7) for determining a magnitude (F) corresponding to the stimulation intensity form the temporal mean value of the stimulation intensity, in which the means (5, 6, 7) for comparing the stimulation intensity compare the mean value of the stimulation intensity with a threshold value (SWF 1, SWF 2) for the mean value of the stimulation intensity and the means (5, 6, 7, 14) for determining a time duration determine that time duration (T 1, T 2) for which the temporal mean value of the stimulation intensity falls below the threshold value (SWF 1, SWF 2) for the mean value of the stimulation intensity.

4. Apparatus according to one of Claims 1 to 3, **characterized in that** it is designed as a cardiac pacemaker, in which the stimulating means (3, 5, 20) stimulate the cardiac activity of the living being and the actuating means (5, 24) for adapting the stimulation intensity adapt the stimulation frequency to the body activity of the living being.

## Revendications

1. Appareil médical implantable dans le corps d'un être vivant, comportant des moyens (3, 5, 20) de stimulation d'un processus physiologique de l'être vivant avec une intensité de stimulation réglable et des moyens de réglage automatique (5, 24) qui sont destinés à adapter l'intensité de stimulation à l'activité corporelle de l'être vivant et qui règlent l'intensité de stimulation au moyen d'un signal, qui correspond à l'activité corporelle de l'être vivant, d'un dispositif de détection (42, 44, 46), la sensibilité (E), avec laquelle s'effectue le réglage de l'intensité de stimulation au moyen du signal du dispositif de détection (42, 44, 46), pouvant être réduite à partir d'une valeur normale (N), **caractérisé par le fait que** l'appareil comporte en outre des moyens (5, 6, 7) de détermination d'une grandeur (F) correspondant à l'intensité de stimulation, des moyens (5, 6, 7) de comparaison de l'intensité de stimulation, qui comparent la grandeur (F) déterminée, qui correspond à l'intensité de stimulation, à une valeur de seuil adéquate (SWF 1), des moyens (5, 6, 7, 14) de détermination de durées, qui déterminent une durée (T 1), pendant laquelle la grandeur (F) qui correspond à l'intensité de stimulation est inférieure à la valeur de seuil (SW 1), et des moyens (5, 6, 7) de comparaison de durées, qui comparent la durée déterminée (T 1) à une durée minimale (SWT 1), un abaissement de la sensibilité (E) ayant lieu, lors d'un dépassement de la durée minimale (SWT 1), à une valeur de sensibilité (E 1) inférieure à la valeur normale (N), et **par le fait que** le dispositif de détection comporte un capteur unique, qui est un capteur (42) de pression piezoélectrique mécaniquement en contact avec le corps de l'être vivant.

2. Appareil suivant la revendication 1, **caractérisé par le fait que** les moyens (5, 6, 7) de comparaison de l'intensité de stimulation comparent la grandeur (F) déterminée, qui correspond à l'intensité de stimulation, à une seconde valeur de seuil (SWF 2), qui est égale ou inférieure à une valeur de seuil (SWF 1), que les moyens (5, 6, 7, 14) de détermination d'une durée déterminent en outre une seconde durée (T 2), pendant laquelle la grandeur (F), qui correspond à l'intensité de stimulation, dépasse la seconde valeur de seuil (SWF 2), et que les moyens (5, 6, 7) de comparaison de durées comparent en outre la seconde durée déterminée (T 2) à une seconde durée minimale (SWT 2), auquel cas, une diminution de la sensibilité (E) ayant lieu, lors d'un dépassement de la seconde durée minimale (SWT 2), à une seconde valeur de sensibilité (E 2) inférieure à la valeur de sensibilité (E 1).

3. Appareil suivant la revendication 1 ou 2, **caractérisé par le fait que** les moyens (5, 6, 7) de détermination d'une grandeur (F) correspondant à l'intensité de stimulation forment la valeur moyenne dans le temps de l'intensité de stimulation, les moyens (5, 6, 7) de comparaison de l'intensité de stimulation comparant la valeur moyenne de l'intensité de stimulation à une valeur de seuil (SWF 1, SWF 2) pour la valeur moyenne de l'intensité de stimulation, et les moyens (5, 6, 7, 14) de détermination d'une durée déterminent la durée (T 1, T 2) pendant laquelle la valeur moyenne dans le temps de l'intensité de stimulation est inférieure à la valeur de seuil (SWF 1, SWF 2) pour la valeur moyenne de l'intensité de stimulation.

4. Appareil suivant l'une des revendications 1 à 3, **caractérisé par le fait qu'**il est agencé sous la forme d'un stimulateur cardiaque, les moyens de stimulation (3, 5, 20) stimulant l'activité cardiaque de l'être vivant, et les moyens de réglage (5, 24), destinés à l'adaptation de l'intensité de stimulation, adaptant la fréquence de stimulation à l'activité corporelle de l'être vivant.
